# EUROPEAN PATENT SPECIFICATION

(11) **EP 3 781 219 B1**
(45) Date of publication and mention of the grant of the patent: **01.06.2022**
(21) Application number: 19719455.8
(22) Date of filing: 15.04.2019
(51) Int. Cl.: A61L 15/08, A61L 15/12, A61L 15/14, A61F 5/058

(54) **A KIT FOR IN SITU IMMOBILIZATION WITH ANTIFUNGAL AND ANTIMICROBIAL EFFECTS**
KIT ZUR IN-SITU-IMMOBILISIERUNG MIT ANTIMYKOTISCHEN UND ANTIMIKROBIELLEN WIRKUNGEN
KIT D'IMMOBILISATION IN SITU AYANT DES EFFETS ANTIFONGIQUES ET ANTIMICROBIENS

(30) Priority: 17.04.2018 HR 20180613; 10.04.2019 HR 20190683
(43) Date of publication of application: 24.02.2021
(73) Proprietor: MP Lumber d.o.o., 10000 Zagreb (HR); Doctors & Co. d.o.o., 43000 Bjelovar (HR)
(72) Inventor: KODVANJ, Janos, 10000 Zagreb (HR); CURIC, Stjepan, 32214 Tordinci (HR)
(74) Representative: Bihar, Zeljko
(86) International application number: PCT/EP2019/059703
(87) International publication number: WO 2019/201872

(56) References cited:
- WO-A1-2006/076932
- WO-A2-2010/103186
- US-A- 5 364 693
- US-A1- 2015 257 918
- US-A1- 2017 058 120

## Description

### Technical Field

The invention relates to immobilization devices and kits containing thereof, which are suitable for *in situ* application. In general, the present invention relates to the field of orthopaedic methods or devices for non-surgical treatment of bones or joints. Specifically, the invention relates to orthopaedic devices, like splints or casts, which harden *in situ* taking a desired form due to the use of thermoplastic materials used for forming the said immobilization devices.

### Technical Problem

Many injuries as well as painful and inflammatory medical conditions require immobilization, which is provided by fixation of the affected body part. Such immobilization decreases the pain of an injured person and enables faster healing of affected tissue structures. Immobilization of body parts can be performed by using various means and materials. In 1852 the Dutch military surgeon Antonius Mathysen first proposed the use of gypsum as a method of immobilizing fractures. Gypsum was added onto the bandage and the formed structure was put into the warm water to form a splint which fitted to the shape of the affected body part, which, after drying and hardening, provided immobilization. Although that was a long time ago, the main principles of gypsum (plaster of Paris) immobilization have not changed significantly. Nowadays, plaster is certainly one of the most commonly used materials for immobilization due to several reasons; it is inexpensive, non-toxic, non-flammable, can be moulded and shaped as required. However, shaping of gypsum is possible only within a short period of time. Once gypsum becomes hard, further forming is not possible. The gypsum technique is very convenient for the repositioning of fragments when the repositioning is done by applying traction or lateral pressure and is to be retained a few minutes after gypsum sets. Advantages in the application are obvious as, except for water, no additional means are necessary.

Besides the above cited advantages, it should be noted that the gypsum immobilization once completed has a significant density, approximately 2.3 g/cm³, which certainly leads to the patients suffering from discomfort and feeling of confinement. In addition, this type of immobilization cannot be immersed into water during the maintenance of patient personal hygiene; it is poorly aerated and slightly prone to crumbling in time. Finally, the medical staff engaged in gypsum immobilization get their hands and workplace permanently dirty.

In the last 30 years there have been major advances in the area of development of novel materials which can be employed for immobilization, and new methods for treatment of fractures have also been introduced. Despite the conservatism in the field, several proposals for new immobilization materials have started to appear, which we will present in a review of the state of the art. The new materials exhibit superior properties; however, they always require longer preparation for their *in-situ* application than the equivalent gypsum immobilization. This longer preparation is a result of the chemical and/or technological nature of the used material, which leads to the situation that the conventional gypsum immobilization of patients remains the first choice in trauma clinics. The article written by J. Hancevic et al. under the title: APPLICATION OF PLASTIC MATERIALS FOR THE PURPOSE OF IMMOBILIZATION [in Croatian], Med. Vjesnik 2000;32(1-4):111-115, available at https://hrcak.srce.hr/index.php?show=clanak&id_clanak_jezik=280789 compares gypsum with modern composite materials used for immobilization.

The first technical problem solved by the present invention is that to perform the immobilization process only water is needed. The proposed novel immobilization kit uses the best from the tradition; i.e. the fact that apart from the components needed for immobilization (traditionally gypsum and bandage) only water is needed, as well as the advantages of existing modern thermoplastic composite materials. It is well known that new thermoplastic composite materials are lighter, water resistant, well aerated and more transparent to X-rays which enables a perfect insight into fragile structures of the bon callus during the phase of bone healing without taking the immobilization off. The latter is not possible with the traditional gypsum-based immobilization. In addition, the proposed kit uses the known advantages of thermoplastic materials, namely, that they become hard very quickly and full load can be immediately applied on them. Working with this kit means that both the medical staff and their workplace can be kept clean.

This first technical problem was solved by designing a kit which consists of three components: a thermoplastic composite, a chemical heater and water or water containing liquid. Water serves as an oxidizing agent for activating the chemical heater which heats up the thermoplastic composite to be fit for the *in situ* forming.

The second technical problem which appears during the *in-situ* application is sterilization; i.e. partial or full sterilization of the immobilization device. As a consequence of solving the first technical problem - heating up of the thermoplastic composite with water and steam - at the same time sterilization of the immobilization device is performed, which is of particular importance when using the kit outside hospitals, e.g. for military purposes on a battlefield. In health institutions possible open wounds will be treated before immobilization, but this is certainly not the case on the front lines. However, in the case of fast immobilization and emergency transport it is important not to worsen the health condition of a patient and to minimise possibilities of contamination of the wound around fractures or other injuries. For this purpose, *in situ* sterilization of the immobilization device represents an obvious advantage thus eliminating the need for applying hygienic standards by both surgeon and patient.

The third technical problem, which occurs in practice, is related to the issue of personal hygiene maintenance of a patient. It is well known that very poor aeration results in the development of fungi and bacteria during the period of immobilization, especially on the contact surface of the immobilization device and the skin of the affected human or animal. It is known that venting channels formed in thermoplastic materials and composites thereof can partially solve the observed problems without compromising the strength of the immobilization device. Although this can be a possible solution to the problem, it only partially avoids the above-mentioned hygiene problems. In the present invention, such materials are used that can easily be mixed with the employed thermoplastic materials and that exhibit proven antifungal and antimicrobial effects, providing prolonged beneficial effects to the patient during the whole time of immobilization. This effect adds up to the effect obtained by solving the second technical problem, which is of short duration, whilst solving this third technical problem represents a long-lasting effect on the patient, human or animal, that was subjected to such a treatment.

### State of the Art

The present invention is oriented to a kit for *in situ* immobilization with antifungal and antimicrobial activity. The review of prior art documents is divided in accordance with the kit components. It starts with the thermoplastic materials and corresponding composite materials used for forming immobilization devices.

Document US 7,985,192B2 for the invention: GEOMETRICALLY APERTURED PROTECTIVE AND/OR SPLINT DEVICE COMPRISING A RE-MOULDABLE THERMOPLASTIC MATERIAL; inventors D. Sheehan et al., teaches about the formation of an immobilization device consisting of thermoplastic poly(ε-caprolactone) with fillers in the form of wood fibres which are added to the said thermoplastic material as a reinforcing agent during the formation of the said device.

Document EP 1845907B1 for the invention: ORTHOSIS AND METHOD FOR MANUFACTURE THEREOF; inventor R. C. V. T. M. J. P. Nieberding, discloses the process for producing orthoses based on the thermoplastic material called ethylene vinyl acetate (EVA) or a similar material, mixed with cork. The formed orthosis comprises apertures with rather large diameters intended for ventilation without compromising the orthosis function.

Document EP 2405951B1 for the invention: NOVEL COMPOSITE MATERIALS COMPRISING A THERMOPLASTIC MATRIX POLYMER AND WOOD PARTICULES; inventors A. Pärssinen et al., teaches about the process for manufacturing a composite material comprising thermoplastic biodegradable polymer such as poly(ε-caprolactone) with small laminar wood pieces as a filler. There is a note in the abstract that, once heated to 60°C, such novel composite can be shaped directly on the patient; i.e. on the patient's skin.

Article available at https://doi.org/10.1016/j.matdes.2015.05.040 and written by E. M. Fernandes et al. under the title: CORK-POLYMER BIOCOMPOSITES: MECHANICAL, STRUCTURAL, AND THERMAL PROPERTIES, Materials & Design 2015;82:282-289, discloses the formation of a composite material comprising cork and low melting point biodegradable polymers such as poly(ε-caprolactone).

From the above review it is evident that composite materials containing thermoplastic materials with added cork and/or other wood components (the filler) are commonly used in the prior art. These kinds of fillers are hereinafter referred to as "organic fillers".

On the other hand, there are also "inorganic fillers" known in the prior art, which can be added to thermoplastic materials to enhance their antifungal and antimicrobial properties. In this sense, the following prior art documents are cited below:
Article available at https://doi.org/10.1016/j.ijpharm.2017.04.077 and written by Z. Muwaffak et al. under the title: PATIENT-SPECIFIC 3D SCANNED AND 3D PRINTED ANTIMICROBIAL POLYCAPROLACTONE WOUND DRESSINGS, Int. J. Pharm. 2017;527(1-2):161-170, describes the addition of silver, copper, and zinc to the above-mentioned poly(ε-caprolactone) (hereinafter referred to as PCL) and 3D printing of orthoses which, due to the used inorganic fillers with antimicrobial activity, enhance wound healing in patients.

Article available at https://doi.org/10.1016/j.eurpolymj.2016.09.028 and written by S. Mallakpour et al. under the title: NANOCOMPOSITES BASED ON BIOSAFE NANO ZnO AND DIFFERENT POLYMERIC MATRIXES FOR ANTIBACTERIAL, OPTICAL, THERMAL AND MECHANICAL APPLICATIONS, Eur. Polymer J. 2016;84:377-403, teaches about antibacterial properties of polymers with added ZnO as an antimicrobial agent.

Article available at https://doi.org/10.1016/j.polymertesting.2015.05.007 and written by J. P. Mofokeng et al. under the title: MORPHOLOGY AND THERMAL DEGRADATION STUDIES OF MELT-MIXED POLY(LACTIC ACID) (PLA)/POLY(E-CAPROLACTONE) (PCL) BIODEGRADABLE POLYMER BLEND NANOCOMPOSITES WITH TiO2 AS FILLER, Polymer Testing 2015;45:93-100, discloses the addition of TiO₂ as a filler to the PCL thermoplastic polymer. It is well known in the art that this inorganic filler exhibits the antimicrobial effect. However, the article highlights the role of TiO₂ in the thermal stability and corresponding TiO₂ degradation prevention properties once the material is embedded into the composite.

Article available at https://doi.org/10.1016/j.biomaterials.2007.02.014 and written by R. De Santis et al. under the title: EFFECTS OF POLYMER AMOUNT AND PROCESSING CONDITIONS ON THE IN VITRO BEHAVIOUR OF HYBRID TITANIUM DIOXIDE/POLYCAPROLACTONE COMPOSITES, Biomaterials 2007;28(18) :2801-2809, teaches about addition of TiO₂ as a filler to the PCL thermoplastic polymer. Besides improved mechanical properties, the investigated composites also showed a very good cytocompatibility as indicated by the MTT assay results presented in the article.

Article available at https://doi.org/10.1016/j.arabjc.2014.12.015 and written by P. Anandgaonker at al. under the title: SYNTHESIS OF TiO2 NANOPARTICLES BY ELECTROCHEMICAL METHOD AND THEIR ANTIBACTERIAL APPLICATION, Arabian Journal of Chemistry 2015, presents TiO₂ nanoparticles and their application in the prevention of human pathogens such as gram-negative *Escherichia coli (E. coli)* and gram-positive species *Staphylococcus aureus.*

Article available at http://www.chalcogen.ro/169_Shoja.pdf and written by M. Shoja et al. under the title: PREPARATION, CHARACTERIZATION AND ANTIBACTERIAL PROPERTIES OF POLYCAPROLACTONE/ZNO MICROCOMPOSITES, Digest J. Nanomaterials and Biostructures 2015;10(1):169-178, teaches about the role of ZnO as an inorganic filler for PCL. The article demonstrates the usefulness of ZnO addition to achieving antimicrobial effects against *Salmonella choleraesuis* as gram-negative bacteria and *Bacillus subtilis* as gram-positive bacteria.

The above listed set of articles show that composite materials selected from among thermoplastic materials, especially PCL, where ZnO and/or TiO₂ are added, are already known in the prior art. The said inorganic fillers provide antimicrobial effects once added to the resulting composites.

It is less known that the previously mentioned organic fillers, e.g. cork, also contribute to antifungal and antimicrobial activity. Article available at https://doi.org/10.1093/femsle/fnv231 and written by F. Goncalves et al. under the title: EVALUATION OF ANTIMICROBIAL PROPERTIES OF CORK, FEMS Microbiol. Lett. 2016;363(3), fnv231, describes the fact that cork exhibits antimicrobial property against *Staphylococcus aureus* with almost 100% efficacy (96.93%) after 90 minutes of incubation. This percentage is significantly lower for *Escherichia coli* being about 36%.

The antifungal and antimicrobial activity results from a complex mixture known as suberin. The antimicrobial and antifungal role of suberin is disclosed in the article available at https://pubs.acs.org/doi/abs/10.1021/bm500201s and written by H. Garcia et al. under the title: EX SITU RECONSTITUTION OF THE PLANT BIOPOLYESTER SUBERIN AS A FILM, Biomacromolecules 2014;15(5):1806-1813. The article states that a suberin film from cork shows a bactericidal property against Gram-positive *(Staphylococcus aureus* NCTC8325) and Gram-negative *(Escherichia coli)* bacteria.

From this review it is evident that composites which consist of thermoplastic materials and carefully selected organic fillers, e.g. cork and other wood particles, and with a possible addition of TiO₂ and/or ZnO will exhibit antimicrobial and antifungal properties. Furthermore, it is evident that the choice of poly(ε-caprolactone) (PCL) enables such an improved composite material to be shaped at temperatures below 100°C and to be directly applied to the skin.

The use of water for engaging chemical heaters, according to this invention, is a preferable way of reaching the temperature required for shaping of the composite used for immobilization and simultaneous activation of suberin and sterilisation by using a steam and a heat.

Document US 5,611,329 for the invention: FLAMELESS HEATER AND METHOD OF MAKING SAME; inventor M. Lamensdorf, teaches about the way of forming a chemical heater suitable for heating meals for military use. In this invention fast-oxidizing magnesium alloys were used with traces of iron as oxidation retardants. Water is hereby used as an oxidizing agent.

Document US 2015/257918 for the invention: THERMOFORMABLE MEDICAL MEMBER WITH HEATER AND METHOD OF MANUFACTURING SAME; inventors A. Laubach et al. teaches about thermoformable splints comprising thermoplastic material such as polycaprolactone, organic filler and water activated heater.

The first synergistic effect is related to the fact that such a cork based composite material exhibits extraordinarily and completely unexpected thermal diffusivity which is not mentioned in any prior art document. The simple experiment reveals that the material consisting of PCL and cork can be taken out from boiling water and, after being wiped dry, it can be directly applicable to a patient with no fear of discomfort and/or burns. The temperature of the said composite can be rather high, close to 95°C, which is far above the temperature reported in the previous document EP2405951B1 (∼60°C) where it is evident that if the temperature is too high, then there is a risk of discomfort or harm to the patient's skin (chapter [0068] of the EP'951); and if the temperature is not high enough, then the material will not be able to properly conform to the patient's body. From this, one can conclude that the thermal diffusivity of the materials used in '951 does not allow a direct and safe application to the patient's skin when heated above 60°C.

To understand how much thermal diffusivity affects the sense of heat it is suggested to study the article written by A. Salazar: ON THERMAL DIFFUSIVITY, Eur. J. Phys. 2003;24(4):351-358, available at http://iopscience.iop.org/article/10.1088/0143-0807/24/4/353/meta.

The second synergistic effect arises from the chemical heater which uses water as oxidizing agent and produces steam which provides sterilization of the immobilization device before its use. Hot steam and hot water at ∼95°C, where the latter is used also as an oxidizing agent, heat up the core of the immobilization device and pass through the core channels (if made) performing the sterilisation of the immobilization device.

The third synergistic effect is the activation of the crystalline aliphatic suberin fraction of cork by the heat. Namely, as noted in the article available at https://www.sciencedirect.com/science/article/pii/S0079670006000682 and written by A. Gandini et al. under the title: SUBERIN: A PROMISING RENEWABLE RESOURCE FOR NOVEL MACROMOLECULAR MATERIALS, Prog. Polym. Sci. 2006;31(10) :878-892, the presence of a crystalline aliphatic suberin fraction in aliphatic lamellae of suberized cells is evident; and this fraction is possible to be activated with hot steam and water.

Finally, the fourth synergistic effect arises from the nature of chemical reactions performed in the chemical heater. In the preferred embodiment, the result of fast oxidation is Mg(OH)₂, which dissolved in water produces alkaline medium. The alkaline medium helps to extract suberin moieties from the cork and other wood particles, such as ferulic acid, that has also antimicrobial properties. The method of suberin moieties extraction is described in the article by A. Gandini et al. already cited above.

From the above review, it can be concluded that the kit consisting of a thermoplastic composite charged with cork particles and heated by a specifically selected chemical heater, represents a non-trivial combination of the prior art with additional synergistic effects.

### Summary of the Invention

The present invention relates to a kit for *in situ* immobilization of humans and animals when immobilization can be done by using water or water-containing liquid only and without other adjuvants. The water serves as an oxidizing agent. The kit minimally comprises:
- one or more immobilization devices;
- at least one chemical heater with a chemical formulation which produces heat in an exothermic reaction with water or water-containing liquid; and
- a container capable of simultaneously enclosing at least one immobilization device and at least one chemical heater.

The immobilization device consists of:
A. a core made of a mixture obtained from the following materials:
   - a thermoplastic material with a melting point Tₘ between 50°C and 100°C;
   - an organic filler selected from among cork particles and other wood particles that lowers the specific weight of the mixture, with a volume percentage from 5% to 60% in the core mixture; and,
   - an optional inorganic filler, selected from among TiO₂, ZnO or their combination which exhibits antimicrobial and antifungal properties, with a weight percentage of up to 25% in the core mixture; and optionally,
B. an additional outer cork layer, with antifungal and antimicrobial properties, which partially or fully covers the surface of the core of the immobilization device, where the thickness of the cork layer is equal to or lower than the lowest thickness of the core of the immobilization device, where the cork layer is bound to the core by the core's thermoplastic material.

The exothermic reaction, started when the chemical heater and water or water-containing liquid come into contact, heats up the immobilization device positioned within the container to temperatures higher than 80°C. The immobilization device is heated not only when it is in contact with the heater but also by the generated hot steam which is accumulated within the container. The generated steam is released through one or more openings made on the container.

The started exothermic reaction brings the core into a plastic state in which it is easily shaped by the surgeon. The released steam and heat sterilize the immobilization device and activate the crystalline aliphatic suberin fraction of cork. The thermal diffusivity coefficient of the immobilization device core, or optionally the thermal diffusivity coefficient of the cork layers, allows direct application of the immobilization device to the human or animal skin without causing discomfort or burns.

In one embodiment, the kit further comprises a water container for water or water-containing liquid as an oxidizing agent in the amount sufficient for activating all chemical heaters.

In another embodiment, the core is manufactured to contain a mesh of planar venting channels and perpendicular venting channels. All or a part of the perpendicular venting channels extend through the cork layers if applied. The venting channels:
(i) promote venting of the human or animal skin surface through the said immobilization device, and
(ii) promote heating of the core by the generated steam and indirectly improve the sterilization of the said immobilization device before its use.

In a preferred embodiment, the chemical formulation is based on fast-corroding alloys, preferably on magnesium alloys and most preferably on alloy Mg - 5% atm. Fe, where the oxidation reaction with magnesium produces heat, steam and Mg(OH)₂, which dissolved in water promote suberin moieties release from wood or cork material used for forming the immobilization device.

In the preferred embodiment, the selected thermoplastic material for the core formation is biodegradable poly(ε-caprolactone). According to another embodiment, the selected organic filler for the core formation consists of cork particles only exhibiting antifungal and antimicrobial properties. In yet another embodiment, the core is formed without an inorganic filler.

In another embodiment, the outer cork layer is absent, and the core is applied directly to the human or animal skin, where the said core exhibits antifungal and antimicrobial properties *per se.*

The present invention discloses the immobilization device with a density less than 1000 kg/m³.

The present invention discloses the method of use of the above described kit wherein the said method includes the following steps:
a) insertion of the immobilization device and the chemical heater into the impermeable container;
b) dosing the water or water-containing liquid into the container up to the filling line marked on the container;
c) leaving the immobilization device and the activated chemical heater within the container be on a flat surface which enables free release of the generated steam through the container opening while the chemical reaction, performed by the chemical heater, brings the core into a plastic state; and
d) immediate application of the immobilization device onto the human or the animal skin, without danger for surgeon or patient.

This immobilization device is suitable for application where necessary that the immobilization device is water resistant and/or the application should produce buoyancy effect for the patient.

### Description of Figures

The present invention and its use are illustrated by the accompanying figures.

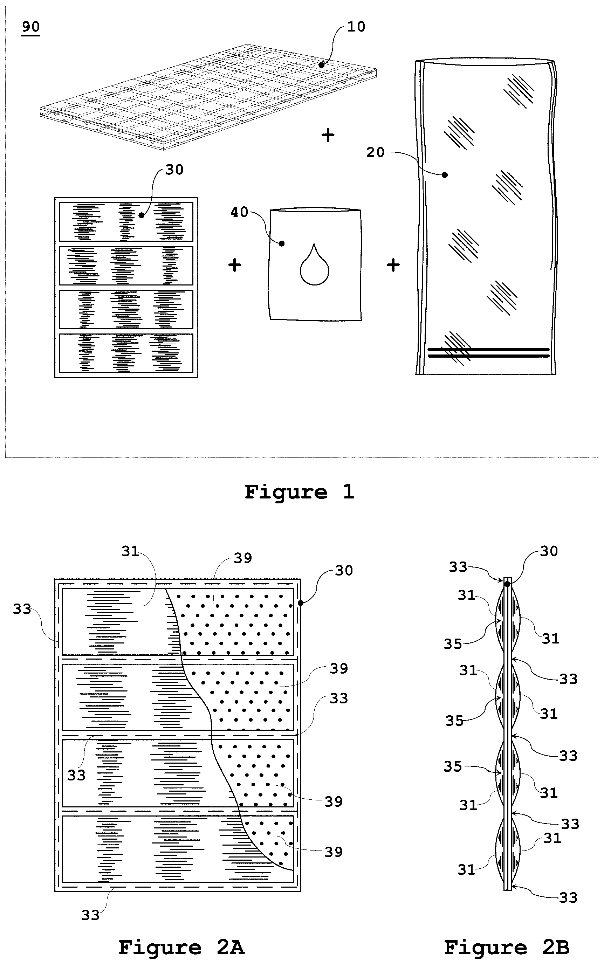

Figure 1 shows the standard kit composition of the present invention. Figures 2A and 2B show the preferred embodiment of the chemical heater.
Figures from 3A to 3H show the procedure for the use of the kit for *in situ* immobilization according to the present invention.

### Detailed Description of the Invention

### 1. The kit

The present invention relates to the kit (90) for *in situ* immobilization of humans and animals as shown in Figure 1. According to the present invention, the kit minimally contains one or more immobilization devices (10), a chemical heater (30) and a container for performing the reaction (20). The container for performing the reaction (20) is the simplest element of the kit (90). The only requirements the container must meet are that it is made of an impermeable material and its size is such that it can enclose at least one immobilization device (10) and at least one chemical heater (30) simultaneously. The kit (90) may further comprise a water container (40) for storing water (50) or a water containing liquid (50) as shown in Figure 1.

### 2. The container

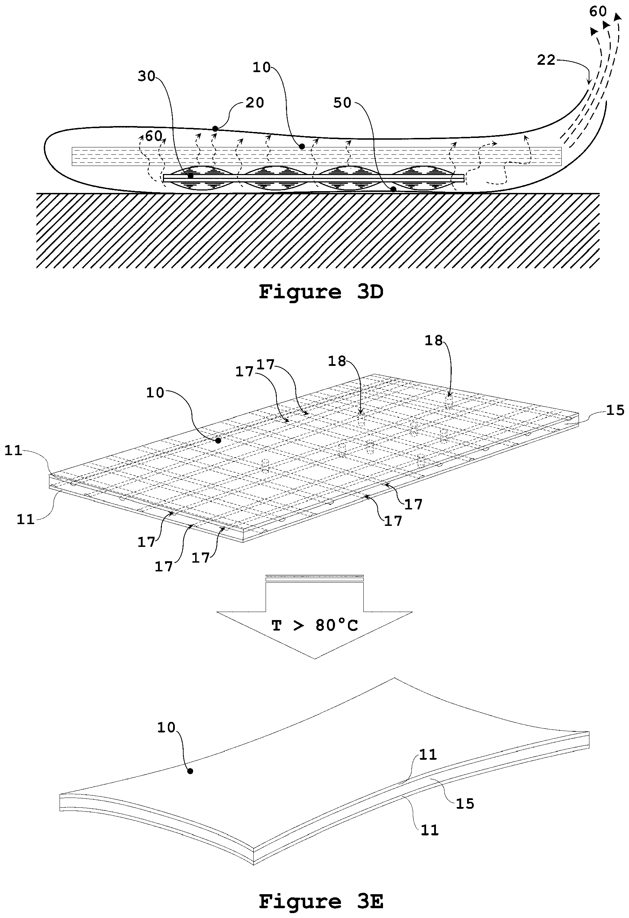

In a preferred embodiment, the container (20) is made of a plastic material having a melting point greater than 120°C, which does not release any toxic substances into water at elevated temperatures. Additionally, the desirable design is that the container (20) is made as a plastic bag with an opening (22) on one side intended for the insertion of the chemical heater (30) and the immobilization device (10) - and with the possibility that water (50) is poured into it up to the marked filling line (21). The filling mark (21) defines the optimal amount of water or water containing liquid (50) required for activating the chemical heater (30), and the use of the whole kit (90). The said opening (22) also serves for removing the generated steam (60) at the same time, as shown in Figure 3D.

### 3. The chemical heater

The structure of the chemical heater (30) is shown in Figure 2A and Figure 2B. The construction is similar or identical to the solution cited in the prior art document US 5,611,329. In the preferred embodiment, the pockets (35) made within the chemical heater (30) are filled with the chemical formulation (39). The pockets (35) have two opposite outer surfaces (31) that are mutually connected. The chemical heater's outer surfaces (31) are connected via seam lines (33), represented by an intermittent line in Figure 2A. The two opposite outer surfaces are connected in any convenient way already known in the prior art; i.e. by ultrasound welding, thermo-bonding, or gluing along the desired portions of the opposite sides (31) - performed along the said lines (33). The material for the outer surfaces (31) is selected in the manner that it is capable of keeping the chemical formulation (39) within the made pockets (35), but it should be permeable to water (50) or water containing liquid (50) used for starting the operation of the chemical heater (30) and to the gases released during the chemical reaction.

In a preferred embodiment, the chemical formulation (39) consists of the following mixture: Mg-Fe alloy, preferably with 5% atm. Fe, NaCl, anti-foaming agent, and internal filler. The surfaces (31) are made of polyester treated with a food-grade surfactant. Polyester surfaces (31) are made gas and water-permeable. It is possible to provide the channels in the design to facilitate the exchange of water and steam within the chemical heater (30) and to speed up the chemical reaction. These channels may allow the water (50) to penetrate to the fast-corroding Mg-Fe alloy even faster, which results in the fastest possible release of heat and steam. Basically, once water or water containing liquid (50) is added, the following chemical reaction occurs:

Mg + 2H₂O -> Mg(OH)₂ + H₂ + heat + steam

A person skilled in the art could use also other types of chemical heaters. The essential requirement is that the degradation products are non-toxic, and that the heater releases the amount of heat and steam (60) necessary to sterilize and soften the immobilization device (10) .

It is clear that the amount of the used chemical formulation (39), intended to be prepared for *in situ* immobilization, depends on the weight of the immobilization device (10). With respect to the above-mentioned chemical reaction of magnesium oxidation, water (50) or some other water containing liquid (50), even body liquid such as urine, which predominantly contains water, is a suitable medium for heating in the preferred embodiment.

### 4. The immobilization device

A lot of information about the immobilization device (10) with antifungal and antimicrobial activity has already been given in the prior art review, however, some additional information about the immobilization device used in the kit (90) is to be provided.

Concerning the fact that the chemical heater (30) is used in a water medium, a person skilled in the art could conclude that the maximum temperature that can be achieved during heating within the container (20) is 100°C due to the water-steam phase transition. At a standard pressure, this transition prevents further heating above the said temperature. Therefore, one must keep in mind that the immobilization device should be plastic at this temperature, as well as at somewhat lower temperatures.

The immobilization device (10), used in the kit (90) with antifungal and antimicrobial effects comprises:
A. a core (15) made of the mixture obtained from the said materials:
   - a thermoplastic material with a melting point Tₘ between 50°C - 100°C;
   - an organic filler selected from among cork particles and other wood particles that lower the specific weight of the mixture, with a volume percentage from 5% to 60% in the core mixture; and,
   - an inorganic filler selected from among TiO₂, ZnO or their combination, which exhibits antimicrobial and antifungal properties, with a weight percentage up to 25% in the core mixture; and optionally,
B. an additional outer cork layer (11), with antifungal and antimicrobial properties, which partially or fully covers the surface of the core (15) of the said immobilization device (10), where the thickness of the cork layer (11) is equal to or lower than the lowest thickness of the core (15) of the said immobilization device, where the said cork layer (11) is bound to the core (15) by the core's thermoplastic material.

The application of the device (10) will be demonstrated on the following examples.

### Example 1

For the core preparation poly(ε-caprolactone) is selected as the best thermoplastic material according to the prior art. The Tₘ of poly(ε-caprolactone) is reported in the literature to be between 58°C and 65°C.

The core (15) is formed without an inorganic filler. In order to amplify the antifungal and antimicrobial effects, an additional cork layer (11) is formed on the external side of the core (15) by thermo-bonding of the core's PCL with the said cork layer (11). Regarding the selection of the cork layer (11) thickness, the diameter of the core (15) must be taken into consideration, i.e. it must bear the cork layer (11) during the shaping of the core (15) in its plastic state, as well as the already used amount of organic fillers that decrease the core (15) thermo-bonding ability.

The organic filler is selected from among cork particles and other wood particles that lower the specific weight of the mixture, and should have a volume percentage from 5% to 60% in the core mixture.

Optionally, the core (15) can be provided with planar venting channels (17) formed once the core (15) is in the plastic state during moulding, or by thermal carving once the core (15) is solid. Perpendicular venting channels (18) may be formed in the same manner, or even once the cork layers (11) are bonded. The perpendicular venting channels (18) may be formed, in an easy manner, by drilling a sandwich structure made of the core (15) and the cork layers (11) as depicted in Figure 3E.

While forming the venting channels, one has to consider the weakening of the core (15) of the said immobilization device (10) by forming the channels (17, 18).

### Example 2

This example is a modification of Example 1, in which the selected organic filler consists only of cork particles which significantly reduce the specific weight. The cork density is 0.2 g/cm³ and the poly(ε-caprolactone) density is around 1.145 g/cm³, from which it is obvious that an immobilization device with a density below 1 g/cm³ (1000 kg/m³) is formed. Optionally, venting channels are made as in Example 1.

### Example 3

Example 3 is a modification of Example 1 in which the core (15) additionally contains inorganic fillers with a weight percentage of up to 25% in the mixture, selected from among TiO₂, ZnO or their combination, which have antimicrobial and antifungal properties. In this embodiment, these inorganic fillers contribute to the strengthening of the core. In this option of the core embodiment, the organic filler is used only to reduce the specific weight. Optionally, venting channels are made as in Example 1.

### Example 4

Example 4 is a modification of Example 3, in which the selected organic filler consists only of cork particles which significantly reduce the specific weight. Optionally, venting channels are made as in Example 1.

### Example 5

For the core preparation poly(ε-caprolactone) is selected again as the best thermoplastic material according to the prior art.

The core (15) is formed without an inorganic filler and without outer additional cork layer (11) but with a high percentage of the organic filler that is selected from among cork particles and other wood particles that lower the specific weight of the mixture, with a volume percentage close to 50% in the core mixture. Having in mind that the primary source of the antifungal and antimicrobial effects now come from the cork particles situated within the core (15), the content of the organic filler has to be significant; possibly it should be entirely made of cork particles. Optionally, venting channels are made as in Example 1.

### Example 6

Example 6 is a modification of Example 5 in which the core (15) additionally contains inorganic fillers with a weight percentage up to 25% in the mixture, selected from among TiO₂, ZnO or their combination, which have antimicrobial and antifungal properties. In this embodiment, these inorganic fillers significantly contribute to the strengthening of the core. In this option of the core embodiment, the organic filler is used only to reduce the specific weight. Optionally, venting channels are made as in Example 1.

The above listed examples are just a demonstration what types of immobilization devices are suitable to be used in this invention.

### Other modifications

It is evident to the person skilled in the art that the general possible choices are:
A. core (15) with or without outer cork layers (11);
B. core (15) with or without inorganic fillers selected;
C. core (15) with corks particles only or mixed with other wooden particles;
where the bonding material is poly(ε-caprolactone) (PCL) used predominantly for forming the core (15) due to its physical properties, which are already well described in the art.

If the technical goal is to produce an immobilization device that is extremely lightweight and water-resistant, the natural choice is the PCL core (15) approx. 2-3 mm in thickness with previously pre-mixed cork particles of 0.5 mm in diameter with a volume percentage up to 50% in the core mixture. Such cores (15) may be provided, optionally, with perpendicular venting channels (18) only with the channel's diameter up to 2 millimetres, and with the surface density of the venting channels of approx. 1 channel per cm².

Additionally, it is clear that the kit (90) can contain one or more immobilization devices (10) as well as one or more chemical heaters (30) for performing *in situ* immobilization, and optionally a water container (40) - all according to the requirements in the field. A great advantage is that nothing, but water is needed to be added to the kit (if not already included into the kit) which makes it useful equally as the gypsum kit with all advantages of usage of composite materials mentioned in the prior art review.

### Industrial Applicability

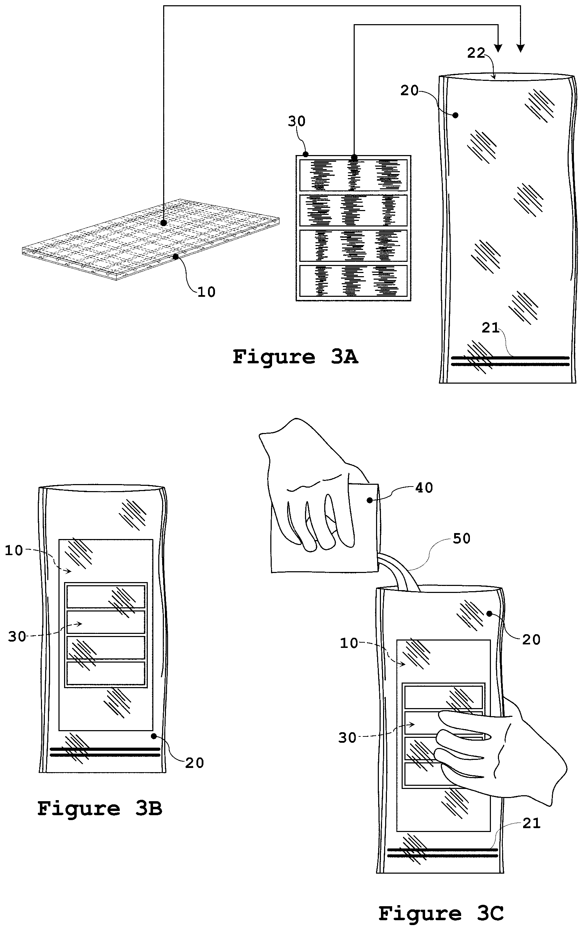

The present invention relates to the use of the kit (90) as such. The use is presented in Figures 3A-3H which demonstrate steps of the standard application.

As shown in Figure 3A, a chemical heater (30) and an immobilization device (10) are inserted into the container (20), which is actually a reinforced plastic bag, as presented in the examples above, and they all form a structure shown in Figure 3B. After that, water (50) as oxidizing agent or a water-containing liquid (50) is poured into the container (20) as shown in Figure 3C, up to the filling line (21). The container (20) is mildly shaken in order to moist the chemical heater (30) and start the exothermic reaction of the formulation (39) of the chemical heater (30) with water. Then, the container is left to sit on a horizontal surface, as shown in Figure 3D. The chemical reaction releases heat and steam (60) which sterilize the immobilization device (10), and come out of the opening (22) on the container (20). If channels (17, 18) are provided on the immobilization device (10), the steam (60) sterilises even more quickly and activates the crystalline aliphatic suberin fraction of cork.

In addition, if Mg(OH)₂ is formed in the reaction, once dissolved in the water it promotes the suberin moieties release from wood or cork material used for forming the immobilization device (10), where the moieties further sterilize the immobilization device (10) as such.

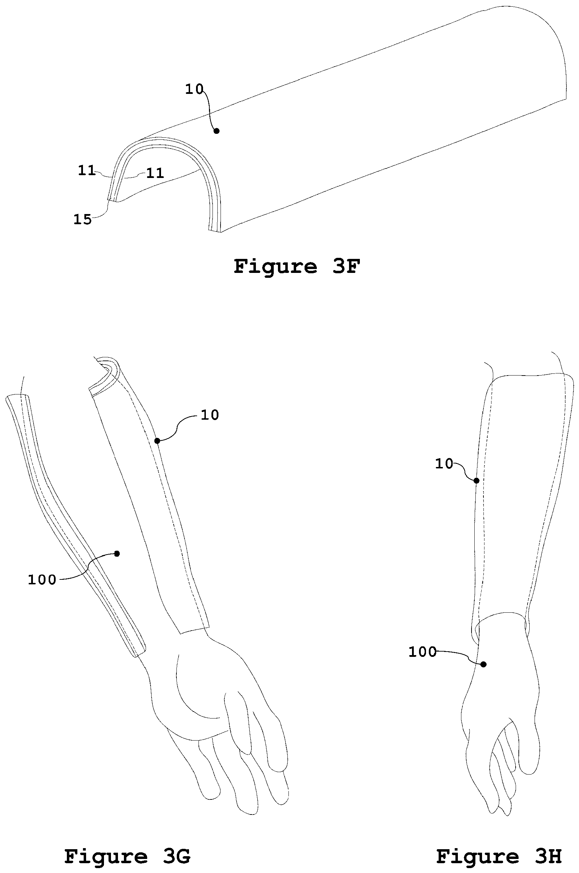

The heating of the container (20) content brings the core (15) of the immobilization device (10) into a plastic state, as shown in Figures 3E and 3F.

Due to a large thermal diffusivity coefficient of such heated materials, the said material is in the plastic state and immediately ready for direct application to the skin (100) of a patient, human or animal, essentially without the step of cooling and without causing discomfort or burns; Figures 3G and 3H. In view of the above-mentioned facts, one can conclude that the industrial applicability of the present invention, in the widest sense of its definition, is obvious.

### Reference numbers

- 10: immobilization device
- 11: outer layer
- 15: core
- 17: planar venting channel
- 18: perpendicular venting channel
- 20: container for performing the reaction
- 21: filling line
- 22: container opening
- 30: chemical heater
- 31: outer surface
- 33: seam line
- 35: pocket filled with chemical mixture
- 39: chemical formulation
- 40: water container
- 50: water or water containing liquid
- 60: steam
- 90: kit
- 100: human or the animal skin

## Claims

1. A kit (90) for *in situ* immobilization of humans and animals, where the said immobilization is possible to be performed by using water or water-containing liquid (50) only and without other adjuvants, where water (50) serves as an oxidizing agent; where the said kit (90) minimally comprises:
- one or more immobilization devices (10) which consists of:
A. a core (15) made of the mixture obtained from:
- a thermoplastic material with a melting point Tₘ between 50°C - 100°C;
- an organic filler selected from cork particles and other wood particles that lower the specific weight of the mixture, with a volume percentage from 5% to 60% in the core (15) mixture;
- an optional inorganic filler; selected from TiO₂, ZnO or their combination and which exhibits antimicrobial and antifungal properties, with a weight percentage up to 25% in the core (15) mixture; and optionally,
B. an additional outer cork layer (11) with antifungal and antimicrobial properties, which partially or fully covers the surface of the core (15) of the said immobilization device (10), where the thickness of the cork layer (11) is equal to or lower than the lowest thickness of the core (15) of the said immobilization device (10), where the said cork layer (11) is bound to the core (15) by the core's thermoplastic material;
- at least one chemical heater (30) with a chemical formulation (39) which produces heat in an exothermic reaction with water or water-containing liquid (50); and
- a container (20);
**characterized by that:**
- the said container is capable of simultaneously enclosing at least one immobilization device (10) and at least one chemical heater (30) which is not part of the immobilization device (10); where the said container (20) is made of impermeable material;
- where an exothermic reaction, started when the chemical heater (30) and the water or water-containing liquid (50) come into contact, heats up the immobilization device (10) positioned within the container (20) to temperatures higher than 80°C in a direct contact of the immobilization device (10) with the heater (30), and additionally with the generated hot steam (60) which accumulates within the container (20); where the said generated steam (60) is released through one or more openings (22) on the container (20);
- wherein the said exothermic reaction brings the core (15) into the plastic state in which it is easily shaped by a surgeon; where the released steam (60) and heat sterilize the said immobilization device (10) and activate the crystalline aliphatic suberin fraction of cork; and
- where the thermal diffusivity coefficient of the immobilization device core (15), or optionally the thermal diffusivity coefficient of the cork layers (11) allows direct application of the immobilization device (10) to the human or animal skin (100) without causing discomfort or burns.

2. A kit (90) for *in situ* immobilization according to claim 1, wherein the said kit (90) comprises a container (40) for water or water-containing liquid (50) as an oxidizing agent in the amount sufficient for starting all chemical heaters (30).

3. A kit (90) for *in situ* immobilization according to any of claims 1 or 2, wherein the core (15) is manufactured to contain a mesh of planar venting channels (17) and perpendicular venting channels (18), where all or a part of the perpendicular venting channels (18) extend through the cork layers (11) if applied; where the said venting channels (17, 18):
(i) promote venting of the human or animal skin surface (100) through the said immobilization device (10), and
(ii) promote heating of the core (15) by the generated steam (60) and indirectly improve the sterilization of the said immobilization device (10) before the use.

4. A kit (90) for *in situ* immobilization according to any of the preceding claims, wherein the chemical formulation (39) is based on fast-corroding alloys, preferably on magnesium alloys and most preferably on the alloy Mg - 5% atm. Fe, where the oxidation reaction with magnesium produces heat, steam (60) and Mg(OH)₂ which, once dissolved in water (50), promotes suberin moieties release from wood or cork material used for forming the immobilization device (10).

5. A kit (90) for *in situ* immobilization according to any of the preceding claims, wherein the selected thermoplastic material for the core (15) formation is biodegradable poly(ε-caprolactone).

6. A kit (90) for *in situ* immobilization according to claim 5, wherein the selected organic filler for the core (15) formation consists of cork particles only, exhibiting antifungal and antimicrobial properties.

7. A kit (90) for *in situ* immobilization according to any of claims 5-6, wherein the core (15) is formed without an inorganic filler.

8. A kit (90) for *in situ* immobilization according to any of claims 5-7, wherein the outer cork layer (11) is absent, and where the core (15) applied directly to the human or animal skin (100) exhibits antifungal and antimicrobial properties *per se.*

9. A kit (90) for *in situ* immobilization according to any of the preceding claims wherein the density of the immobilization device (10) is less than 1000 kg/m³.

10. Kit (90) for *in situ* immobilization defined by any of claims 1-9 for use in a method including the following steps:
a) insertion of the immobilization device (10) and the chemical heater (30) into the impermeable container (20);
b) dosing the water or water-containing liquid (50) into the container (20) up to the filling line (21) marked on the container (20);
c) leaving the immobilization device (10) and the activated chemical heater (30) within the container (20), on a flat surface to enable free release of the generated steam (60) through the container openings (22) while the chemical reaction, performed by the said chemical heater (30), brings the core (15) into the plastic state; and
d) an immediate application of the immobilization device (10) onto the human or the animal skin (100), without any danger for surgeon or patient.

11. Kit (90) for *in situ* immobilization according to any of claims 1-9, wherein the immobilization device (10) is water resistant and the application produce buoyancy effect for the patient.

## Patentansprüche

1. Kit (90) zur In-Situ-Immobilisierung von Menschen und Tieren, wobei Durchführung der Immobilisierung unter ausschließlicher Verwendung von Wasser oder wasserhaltiger Flüssigkeit (50) und ohne anderen Zusatzstoffen möglich ist, wobei Wasser (50) als ein Oxidationsmittel dient; wobei der Kit (90) minimal umfasst:
- eine oder mehrere Immobilisierungsvorrichtungen (10), die aus Folgendem besteht:
A. einem Kern (15), der aus einem Gemisch hergestellt ist, das erhalten wird aus:
- einem thermoplastischen Material mit einem Schmelzpunkt Tₘ zwischen 50 °C - 100 °C;
- einem organischen Füllstoff, ausgewählt aus Korkpartikeln und anderen Holzpartikeln, die das spezifische Gewicht des Gemischs senken, mit einem Volumenprozentsatz von 5% bis 60% im Gemisch des Kerns (15);
- einem optionalen anorganischen Füllstoff; ausgewählt aus TiO₂, ZnO oder deren Kombinationen und der antimikrobielle und antimykotische Eigenschaften aufweist, mit einem Gewichtsprozentsatz von bis zu 25% im Gemisch des Kerns (15);
und optional,
B. einer zusätzlichen äußeren Korkschicht (11) mit antimykotischen und antimikrobiellen Eigenschaften, die die Oberfläche des Kerns (15) der Immobilisierungsvorrichtung (10) teilweise oder vollständig abdeckt, wobei die Dicke der Korkschicht (11) gleich oder kleiner als die kleinste Dicke des Kerns (15) der Immobilisierungsvorrichtung (10) ist, wobei die Korkschicht (11) durch das thermoplastische Material des Kerns an den Kern (15) gebunden ist;
- mindestens ein chemisches Heizmittel (30) mit einer chemischen Formulierung (39), die Hitze in einer exothermen Reaktion mit Wasser oder wasserhaltiger Flüssigkeit (50) erzeugt; und
- einen Behälter (20);
**dadurch gekennzeichnet, dass:**
- der Behälter imstande ist, mindestens eine Immobilisierungsvorrichtung (10) und mindestens ein chemisches Heizmittel (30), das nicht Teil der Immobilisierungsvorrichtung (10) ist, gleichzeitig zu umschließen; wobei der Behälter (20) aus undurchlässigem Material hergestellt ist;
- wobei eine exotherme Reaktion, die startet, wenn das chemische Heizmittel (30) und das Wasser oder die wasserhaltige Flüssigkeit (50) in Kontakt treten, die Immobilisierungsvorrichtung (10), die innerhalb des Behälters (20) platziert ist, in einem direkten Kontakt der Immobilisierungsvorrichtung (10) mit dem Heizmittel (30), und zusätzlich mit dem erzeugten heißen Dampf (60), der sich innerhalb des Behälters (20) ansammelt, auf Temperaturen höher als 80 °C erhitzt; wobei der erzeugte Dampf (60) durch eine oder mehrere Öffnungen (22) am Behälter (20) freigelassen wird;
- wobei die exotherme Reaktion den Kern (15) in den plastischen Zustand versetzt, in dem er leicht von einem Chirurgen geformt werden kann; wobei der freigelassene Dampf (60) und Hitze die Immobilisierungsvorrichtung (10) sterilisieren und den kristallinen aliphatischen suberinen Korkanteil aktivieren; und
- wobei der Koeffizient thermischer Diffusionsfähigkeit des Kerns (15) der Immobilisierungsvorrichtung oder optional der Koeffizient thermischer Diffusionsfähigkeit der Korkschichten (11) direktes Anlegen der Immobilisierungsvorrichtung (10) an die menschliche oder tierische Haut (100) ermöglicht, ohne Beschwerden oder Verbrennungen zu verursachen.

2. Kit (90) zur In-Situ-Immobilisierung nach Anspruch 1, wobei der Kit (90) einen Behälter (40) für Wasser oder wasserhaltige Flüssigkeit (50) als ein Oxidationsmittel in der Menge, die ausreicht, um alle chemischen Heizmittel (30) zu starten, umfasst.

3. Kit (90) zur In-Situ-Immobilisierung nach einem der Ansprüche 1 oder 2, wobei der Kern (15) so gefertigt ist, dass er ein Netz von ebenen Belüftungskanälen (17) und senkrechten Belüftungskanälen (18) enthält, wobei alle oder ein Teil der senkrechten Belüftungskanäle (18) sich durch die Korkschichten (11) erstrecken, falls angelegt; wobei die Belüftungskanäle (17, 18):
(i) Belüftung der menschlichen oder tierischen Hautoberfläche (100) durch die Immobilisierungsvorrichtung (10) hindurch unterstützen, und
(ii) Erhitzen des Kerns (15) durch den erzeugten Dampf (60) unterstützen und die Sterilisierung der Immobilisierungsvorrichtung (10) vor der Verwendung indirekt verbessern.

4. Kit (90) zur In-Situ-Immobilisierung nach einem der vorstehenden Ansprüche, wobei die chemische Formulierung (39) auf schnell korrodierende Legierungen, vorzugsweise auf Magnesiumlegierungen und besonders bevorzugt auf der Legierung Mg - 5% atm. Fe basiert, wobei die Oxidationsreaktion mit Magnesium Hitze, Dampf (60) und Mg(OH)₂ erzeugt, das, nachdem es in Wasser (50) aufgelöst ist, die Lösung von suberinen Teilen aus Holz- oder Korkmaterial, das zur Bildung der Immobiliserungsvorrichtung (10) verwendet wird, unterstützt.

5. Kit (90) zur In-Situ-Immobilisierung nach einem der vorstehenden Ansprüche, wobei das für die Bildung des Kerns (15) ausgewählte thermoplastische Material biologisch abbaubares Poly(ε-caprolacton) ist.

6. Kit (90) zur In-Situ-Immobilisierung nach Anspruch 5, wobei der ausgewählte organische Füllstoff für die Bildung des Kerns (15) ausschließlich aus Korkpartikeln besteht, die antimykotische und antimikrobielle Eigenschaften aufweisen.

7. Kit (90) zur In-Situ-Immobilisierung nach einem der Ansprüche 5-6, wobei der Kern (15) ohne einen anorganischen Füllstoff gebildet wird.

8. Kit (90) zur In-Situ-Immobilisierung nach einem der Ansprüche 5-7, wobei die äußere Korkschicht (11) fehlt und wobei der Kern (15), der direkt an die menschliche oder tierische Haut (100) angelegt wird, *an sich* antimykotische und antimikrobielle Eigenschaften aufweist.

9. Kit (90) zur In-Situ-Immobilisierung nach einem der vorstehenden Ansprüche, wobei die Dichte der Immobilisierungsvorrichtung (10) geringer als 1000 kg/m³ ist.

10. Kit (90) zur In-Situ-Immobilisierung, wie in einem der Ansprüche 1-9 definiert, zur Verwendung in einem Verfahren, das die folgenden Schritte einschließt:
a) Einsetzen der Immobilisierungsvorrichtung (10) und des chemischen Heizmittels (30) in den undurchlässigen Behälter (20);
b) Dosieren des Wassers oder der wasserhaltigen Flüssigkeit (50) in den Behälter (20) bis zur Abfülllinie (21), die auf dem Behälter (20) markiert ist;
c) Belassen der Immobilisierungsvorrichtung (10) und des aktivierten chemischen Heizmittels (30) innerhalb des Behälters (20) auf einer flachen Oberfläche, um ungehindertes Freilassen des erzeugten Dampfes (60) durch die Behälteröffnungen (22) zu ermöglichen, während die vom chemischen Heizmittel (30) durchgeführte chemische Reaktion den Kern (15) in den plastischen Zustand versetzt; und
d) ein unmittelbares Anlegen der Immobilisierungsvorrichtung (10) an die menschliche oder die tierische Haut (100), ohne jegliche Gefahr für Chirurg oder Patient.

11. Kit (90) zur In-Situ-Immobilisierung nach einem der Ansprüche 1-9, wobei die Immobilisierungsvorrichtung (10) wasserbeständig ist und das Anlegen eine Auftriebswirkung für den Patienten bewirkt.

## Revendications

1. Kit (90) pour une immobilisation in situ d'humains et d'animaux, où ladite immobilisation est possible pour être réalisée à l'aide d'eau ou d'un liquide contenant de l'eau (50) uniquement et sans autres adjuvants, où l'eau (50) sert d'agent oxydant; où ledit kit (90) comprend au minimum :
- un ou plusieurs dispositifs d'immobilisation (10) qui se composent de :
A. un noyau (15) constitué du mélange obtenu à partir :
- d'un matériau thermoplastique présentant un point de fusion Tₘ entre 50 °C et 100 °C ;
- d'une charge organique choisie parmi des particules de liège et d'autres particules de bois qui abaissent le poids spécifique du mélange, avec un pourcentage en volume de 5 % à 60 % dans le mélange de noyau (15) ;
- d'une charge inorganique facultative ; choisie parmi TiO₂, ZnO ou leur combinaison et qui présente des propriétés antimicrobiennes et antifongiques, avec un pourcentage en poids allant jusqu'à 25 % dans le mélange de noyau (15) ;
et facultativement,
B. une couche de liège externe additionnelle (11) avec des propriétés antifongiques et antimicrobiennes, qui recouvre partiellement ou totalement la surface du noyau (15) dudit dispositif d'immobilisation (10), où l'épaisseur de la couche de liège (11) est égale ou inférieure à l'épaisseur la plus faible du noyau (15) dudit dispositif d'immobilisation (10), où ladite couche de liège (11) est liée au noyau (15) par le matériau thermoplastique du noyau ;
- au moins un dispositif de chauffage chimique (30) avec une formulation chimique (39) qui produit de la chaleur lors d'une réaction exothermique avec de l'eau ou un liquide contenant de l'eau (50) ; et
- un récipient (20) ;
**caractérisé en ce que** :
- ledit récipient est capable d'enfermer simultanément au moins un dispositif d'immobilisation (10) et au moins un dispositif de chauffage chimique (30) qui ne fait pas partie du dispositif d'immobilisation (10) ; où ledit récipient (20) est constitué d'un matériau imperméable ;
- où une réaction exothermique, démarrée lorsque le dispositif de chauffage chimique (30) et l'eau ou le liquide contenant de l'eau (50) entrent en contact, chauffe le dispositif d'immobilisation (10) positionné à l'intérieur du récipient (20) jusqu'à des températures supérieures à 80 °C dans un contact direct du dispositif d'immobilisation (10) avec le dispositif de chauffage (30), et en outre avec la vapeur chaude générée (60) qui s'accumule au sein du récipient (20) ; où ladite vapeur générée (60) est libérée à travers une ou plusieurs ouvertures (22) sur le récipient (20) ;
- dans lequel ladite réaction exothermique amène le noyau (15) dans l'état plastique où il est facilement mis en forme par un chirurgien ; où la vapeur libérée (60) et la chaleur stérilisent ledit dispositif d'immobilisation (10) et activent la fraction subérine aliphatique cristalline du liège ; et
- où le coefficient de diffusivité thermique du noyau de dispositif d'immobilisation (15), ou facultativement le coefficient de diffusivité thermique des couches de liège (11) permet une application directe du dispositif d'immobilisation (10) sur la peau humaine ou animale (100) sans provoquer de gêne ou de brûlures.

2. Kit (90) pour une immobilisation in situ selon la revendication 1, dans lequel ledit kit (90) comprend un récipient (40) pour de l'eau ou un liquide contenant de l'eau (50) en tant qu'agent oxydant dans la quantité suffisante pour démarrer tous les dispositifs de chauffage chimique (30).

3. Kit (90) pour une immobilisation in situ selon l'une quelconque des revendications 1 ou 2, dans lequel le noyau (15) est fabriqué pour contenir un maillage de canaux d'aération planaires (17) et de canaux d'aération perpendiculaires (18), où la totalité ou une partie des canaux d'aération perpendiculaires (18) s'étendent à travers les couches de liège (11) en cas d'application ; où lesdits canaux d'aération (17, 18) :
(i) favorisent l'aération de la surface de peau humaine ou animale (100) à travers ledit dispositif d'immobilisation (10), et
(ii) favorisent le chauffage du noyau (15) par la vapeur générée (60) et améliorent indirectement la stérilisation dudit dispositif d'immobilisation (10) avant l'utilisation.

4. Kit (90) pour une immobilisation in situ selon l'une quelconque des revendications précédentes, dans lequel la formulation chimique (39) est basée sur des alliages à corrosion rapide, de préférence des alliages de magnésium et le plus préférentiellement de l'alliage Mg-Fe à atm à 5 %, où la réaction d'oxydation avec le magnésium produit de la chaleur, de la vapeur (60) et du Mg(OH)₂ qui, une fois dissous dans l'eau (50), favorise la libération de fractions subérine à partir d'un matériau en bois ou en liège utilisé pour former le dispositif d'immobilisation (10).

5. Kit (90) pour une immobilisation in situ selon l'une quelconque des revendications précédentes, dans lequel le matériau thermoplastique choisi pour la formation de noyau (15) est une poly(ε-caprolactone) biodégradable.

6. Kit (90) pour une immobilisation in situ selon la revendication 5, dans lequel la charge organique choisie pour la formation de noyau (15) est composée de particules de liège uniquement, présentant des propriétés antifongiques et antimicrobiennes.

7. Kit (90) pour une immobilisation in situ selon l'une quelconque des revendications 5-6, dans lequel le noyau (15) est formé sans charge inorganique.

8. Kit (90) pour une immobilisation in situ selon l'une quelconque des revendications 5-7, dans lequel la couche de liège externe (11) est absente, et où le noyau (15) appliqué directement sur la peau humaine ou animale (100) présente des propriétés antifongiques et antimicrobiennes *en soi.*

9. Kit (90) pour une immobilisation in situ selon l'une quelconque des revendications précédentes, dans lequel la densité du dispositif d'immobilisation (10) est inférieure à 1000 kg/m³.

10. Kit (90) pour une immobilisation in situ définie par l'une quelconque des revendications 1-9 pour une utilisation dans un procédé incluant les étapes suivantes :
a) insertion du dispositif d'immobilisation (10) et du dispositif de chauffage chimique (30) dans le récipient imperméable (20) ;
b) dosage de l'eau ou du liquide contenant de l'eau (50) dans le récipient (20) jusqu'à la ligne de remplissage (21) marquée sur le récipient (20) ;
c) le fait de laisser le dispositif d'immobilisation (10) et le dispositif de chauffage activé (30) au sein du récipient (20) sur une surface plate pour permettre une libération sans entraves de la vapeur générée (60) à travers les ouvertures de récipient (22) pendant la réaction chimique, réalisée par ledit dispositif de chauffage chimique (30), amène le noyau (15) dans l'état plastique ; et
d) une application immédiate du dispositif d'immobilisation (10) sur la peau humaine ou animale (100), sans aucun danger pour le chirurgien ou le patient.

11. Kit (90) pour une immobilisation in situ selon l'une quelconque des revendications 1-9, dans lequel le dispositif d'immobilisation (10) est résistant à l'eau et l'application produit un effet de flottabilité pour le patient.
